(19) Europäisches Patentamt
European Patent Office
Office européen des brevets

(11) Veröffentlichungsnummer: **0 210 342 A2**

# (12) EUROPÄISCHE PATENTANMELDUNG

(21) Anmeldenummer: 86105280.1

(22) Anmeldetag: 16.04.86

(51) Int. Cl.⁴: **A61K 31/47**, A61K 31/505

Die Bezeichnung der Erfindung wurde geändert (Richtlinien für die Prüfung im EPA, A-III, 7.3).

(30) Priorität: 03.05.85 DE 3515882

(43) Veröffentlichungstag der Anmeldung: 04.02.87 Patentblatt 87/06

(84) Benannte Vertragsstaaten: BE CH DE FR GB IT LI LU NL SE

(71) Anmelder: **Dr. Karl Thomae GmbH**
Postfach 1755
D-7950 Biberach (Riss)(DE)

(72) Erfinder: **Dämmgen, Jürgen, Dr.**
Eichweg 7
D-7951 Maselheim/Sulmingen(DE)
Erfinder: **Ballhause, Helmut**
Fohrenweg 6
D-7950 Biberach 1(DE)
Erfinder: **Haarmann, Walter, Dr.**
Schlierholzweg 27
D-7950 Biberach 1(DE)
Erfinder: **Heider, Joachim, Dr. Dipl.-Chem.**
Am Hang 3
D-7951 Warthausen 1(DE)

(54) **Arzneimittel, enthaltend Pyridinone mit antithrombotischer Wirkung.**

(57) Die Erfindung betrifft die neue Verwendung der Verbindungen der allgemeinen Formel

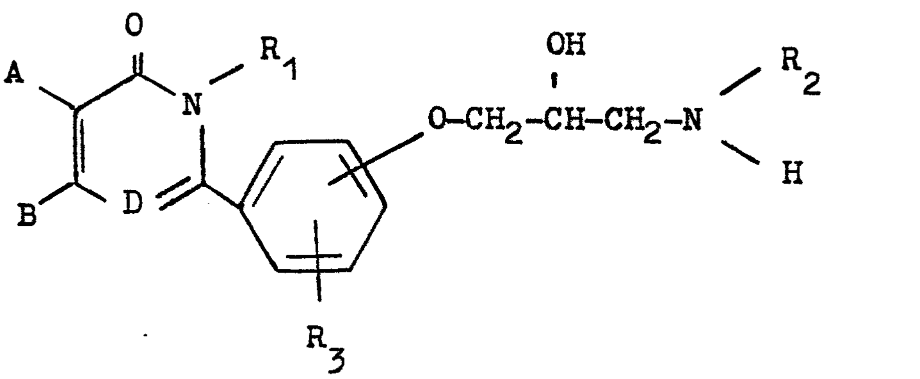
,(I)

in der

A und B zusammen mit den beiden dazwischenliegenden Kohlenstoffatomen einen Methoxyphenyl-, Dimethoxyphenyl-oder Pyridinring,

D ein Stickstoffatom oder eine CH-Gruppe,

$R_1$ ein Wasserstoffatom oder eine Methylgruppe,

$R_2$ eine verzweigte Alkylgruppe mit 3 bis 6 Kohlenstoffatomen oder eine geradkettige Alkylengruppe mit 2 oder 3 Kohlenstoffatomen, die endständig

EP 0 210 342 A2

durch eine Methoxyphenyl-, Dimthoxyphenyl-oder Methylphenoxygruppe substituiert ist, und

$R_3$ ein Wasserstoffatom oder eine Methoxygruppe bedeuten, deren Antipoden und deren physiologisch verträgliche Säureadditionssalze mit anorganischen oder organischen Säuren zur Behandlung und zur Prophylaxe thrombo-embolischer Erkrankungen, zur Prophylaxe der Arteriosklerose und zur Metastasenprophylaxe.

# Arzneimittel, enthaltend Pyridinone mit antithrombotischen Wirkungen und Verfahren zu ihrer Herstellung

In der BE-A-863.160 und in der EP-A-0.054.132 werden Pyridinone der allgemeinen Formel

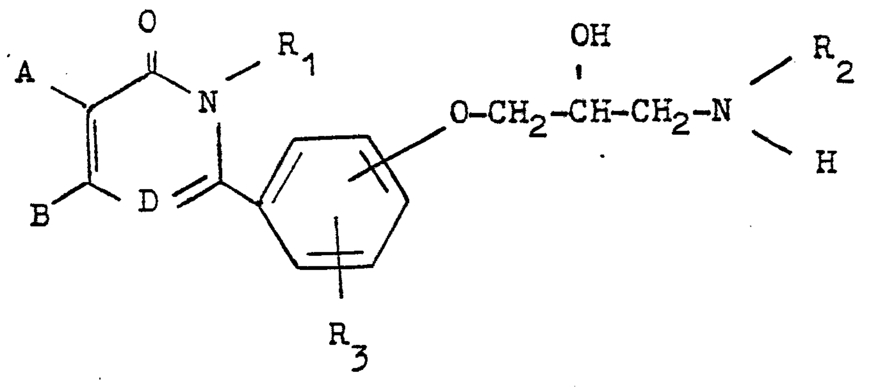

,(I)

in der

A und B zusammen mit den beiden dazwischenliegenden Kohlenstoffatomen einen Methoxyphenyl-, Dimethoxyphenyl-oder Pyridinring,

D ein Stickstoffatom oder eine CH-Gruppe,

$R_1$ ein Wasserstoffatom oder eine Methylgruppe,

$R_2$ eine verzweigte Alkylgruppe mit 3 bis 6 Kohlenstoffatomen oder eine geradkettige Alkylengruppe mit 2 oder 3 Kohlenstoffatomen, die endständig durch eine Methoxyphenyl-, Dimethoxyphenyl-oder Methylphenoxygruppe substituiert ist, und

$R_3$ ein Wasserstoffatom oder eine Methoxygruppe bedeuten, und deren physiologisch verträgliche Säureadditionssalze mit anorganischen oder organischen Säuren beschrieben, welche wertvolle pharmakologische Eigenschaften aufweisen, insbesondere eine antianginöse, antiarrhythmische, blutdrucksenkende und β-Rezeptoren-blockierende Wirkung.

Überraschenderweise wurde nun gefunden, daß die Verbindungen der allgemeinen Formel I sowie deren Antipoden und deren physiologisch verträgliche Säureadditionssalze bei einer Dosierung von 0,3 bis 4 mg/kg Körpergewicht, vorzugsweise von 0,3 bis 2 mg/kg Körpergewicht, ein-bis viermal täglich antithrombotische Eigenschaften aufweisen.

Für die bei der Definition des Restes $R_2$ eingangs erwähnten Bedeutungen kommt beispielsweise die Bedeutung der Isopropyl-, Isobutyl-, tert.Butyl-, Neopentyl-, tert.Pentyl-, Isoamyl-, 3-Methyl-n-pentyl-, 2-(Methoxyphenyl)-äthyl-, 3-(Methoxyphenyl)-n-propyl-, 2-(Dimethoxyphenyl)-äthyl-, 3-(Dimethoxyphenyl)-n-propyl-, 2-(Methoxyphenoxy)-äthyl-oder 2-(Methylphenoxy)-n-propylgruppe in Betracht.

Bevorzugte Verbindungen sind jedoch die Verbindungen der allgemeinen Formel

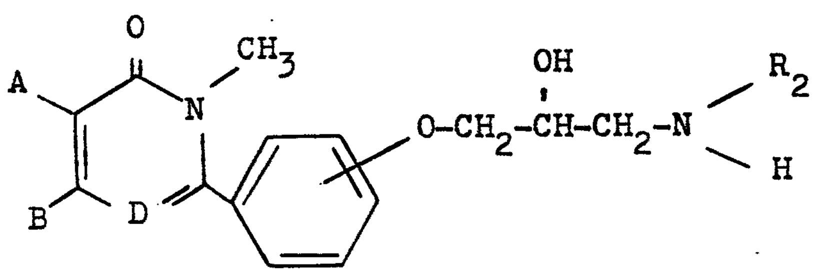

,(II)

in der

D wie eingangs definiert ist,

A und B zusammen mit den beiden dazwischenliegenden Kohlenstoffatomen einen Methoxyphenyl-, Dimethoxyphenyl-oder Pyridinring und $R_2$ eine Isopropyl-oder tert.Butylgruppe darstellen, insbesondere diejenigen Verbindungen, in denen A und B zusammen mit den beiden dazwischenliegenden Kohlenstoffatomen eine

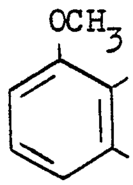 , 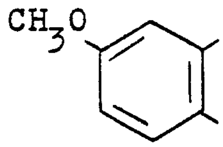 , 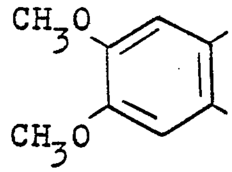 oder 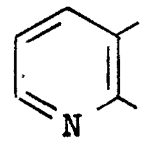 -Gruppe

bedeutet, deren Antipoden und deren physiologisch verträgliche Säureadditionssalze.

Beispielsweise wurden die antithrombotischen Wirkungen der Verbindungen

A = 2-Methyl-3-[4-(2-hydroxy-3-tert.butylamino-propoxy)-phenyl]-7-methoxy-isochinolin-1-(2H)-on,

B = 2-[4-(2-Hydroxy-3-tert.butylamino-propoxy)-phenyl]-3-methyl-6-methoxy-3,4-dihydro-chinazolin-4-on und

C = (-)-2-2[4-(2-Hydroxy-3-tert.butylamino-propoxy)-phenyl]-3-methyl-6-methoxy-3,4-dihydro-chinazolin-4-on wie folgt geprüft:

Hunde beiderlei Geschlechts (Mischrassige Hunde, Körpergewicht 18-35 kg) wurden mit Morphin 1 mg/kg s.c. prämediziert und mit Pentobarbital 30 mg/kg i.v. narkotisiert. Die Narkose wurde durch eine Dauerinfusion mit Pentobarbital ca. 6 mg/kg/h aufrechterhalten. Die Körpertemperatur wurde mit einem Heizkissen und einer Regeleinheit bei 37°C gehalten.

Nach intratrachealer Intubation wurden die Tiere mit einem Harvard Respirator mit Raumluft so beatment, daß physiologische Blut-$pO_2$ und -pH-Werte erhalten wurden.

Über eine A.fermoralis wurde unter fluoroskopischer Kontrolle ein Millar-Doppeldruck Katheter zur Messung des Aortendruckes und des linksventikulären Druckes eingeführt. Aus dem ventrikulären Drucksignal wurden dp/dtmax und Herzfrequenz kontinuierlich bestimmt.

Der Thorax wurde im fünften linken Interkostalraum eröffnet und das Herz im eröffneten Perikard angehoben. Der Ramus circumflexus der linken Koronararterie wurde in seinem proximalen Bereich freipräpariert und ein passender elektromagnetischer Flowmeßkopf (Statham) angelegt. Unmittelbar hinter der Meßstelle wurde mittels einer durch eine Schraube verstellbaren Klemme aus Leichtmetall das Gefäß stenosiert bis sich spontane Flußschwankungen ausbildeten (Thrombusformationen).

Danach wurde das Gefäß so lange durch Quetschung mit einer Arterienklemme oder hin-und herrollen zwischen zwei Fingerspitzen so stark geschädigt, daß sich die Thromben nicht mehr spontan sondern nur noch durch leichtes Bewegen der Stenoseklemme lösten ('shake loose' nach Folts).

Nach einer 30minütigen Kontrollperiode mit regelmäßigen Thrombusbildungen wurden die Prüfsubstanzen gegeben und die Thrombusbildung für jeweils zwei weitere 30minütige Perioden beobachtet.

Alle Substanzen wurden mit 1 mg/kg i.v. appliziert.

Die Verbindungen A bis C und Atenolol wurden aequimolar in wässriger Weinsäure gelöst. Propranolol-HCl wurde in 0.9 % NaCl gelöst. Als Lösungsmittelkontrolle diente 0.9 % NaCl.

Alle Parameter wurden auf einer IFD Registriereinheit erfaßt.

Die nachfolgenden Tabellen 1 und 2 enthalten die gefundenen Daten:

Tabelle 1:

Fluß im Ramus circumflexus der Arteria coronaria sinistra (ml/min) x ± SE

| Substanz | Zahl der Tiere | Vorwert | Nach Stenose |
|---|---|---|---|
| Kontrolle | 8 | 52 ± 5 | 25 ± 3 |
| Verbindung B | 5 | 32 ± 8 | 15 ± 4 |
| Verbindung C | 4 | 26 ± 6 | 19 ± 3 |
| Verbindung A | 5 | 38 ± 5 | 24 ± 4 |
| Atenolol | 5 | 27 ± 5 | 15 ± 3 |
| Propranolol | 3 | 46 ± 5 | 23 ± 4 |

Tabelle 2:

Anzahl der spontanen Thrombosen in dem Ramus circumflexus der A.coronaria sinistra pro 30 min. x ± SE.

| Substanz | Zahl der Tiere | Vorwert | 0-30 min nach Substanzgabe | 30-60 min | 60-90min |
|---|---|---|---|---|---|
| Kontrolle | 8 | 8.3±0.8 | 8.3±0.8 | 8.3±0.9 | 7.5±1.8 |
| Verbindung C | 4 | 5.5±0.6 | 2.5±0.5* | 1.5±1.0 | |
| Verbindung B | 5 | 5.0±0.7 | 3:2±1.1* | 2.4±1.1* | |
| Verbindung A | 5 | 8.6±0.9 | 6.8±1.9 | 6.5±2.5 | |
| Atenolol | 5 | 7.6±1.0 | 6.8±1.1 | 6.8±1.0 | |
| Propranolol | 3 | 10.3±1.8 | 9.6±1.2 | 9.3±1.5 | |

* $p < 0.05$ im Vergleich zum Vorwert (gepaarter t-test nach Student)

Ergebnisse:

Ca. 60 % der untersuchten Tiere zeigten spontane Thrombusbildung. Diese Häufigkeit entspricht der in der von Bush et al (Circulation 69, 1161-1170, 1984) genannten Häufigkeit von ca. 68 %.

Die in einigen Fällen nach dem Versuch eröffneten Koronargefäße zeigten Risse in der Intima sowie Auflagerungen von rötlich-weißem thrombotischen Material.

Die mittleren arteriellen Drucke, dp/dtmax und Herzfrequenz waren in den einzelnen Gruppen etwa gleich.

Zwischen den Ausgangsflußwerten im Ramus circumflexus der linken Koronararterie bestanden beträchtliche Unterschiede, die z.T. durch die erheblichen Unterschiede der Größe der Versuchstiere erklärt werden können.

Die mittleren Ausgangsflüsse wurden im Mittel der Versuche durch die Stenose um 42 ± 2 % vermindert (z. Vergl. Bush et al Circulation 69 , 1984, 35 bzw. 42 % und Bush et al Circ. Res. 55, 642-652, 1984, 40 %).

Die Zahl der Spontanverschlüsse in der Koronararterie lag zwischen 5 und 10 pro 30 min. Die individuellen Verschlußraten blieben über einen Zeitraum von mindestens 1.5 Stunden sowohl bei den Lösungsmittelkontrollen als auch bei den mit nichtantithrombotisch wirksamen Substanzen behandelten Tieren weitgehend konstant (Tab. 2). Dieser Befund entspricht dem von Bush et al (Cir. Res. 55, 1984), die eine Konstanz in der Frequenz der spontanen Thrombusbildung für mehr als 2 Stunden zeigen konnten.

Verbindung B verminderte die Häufigkeit der Spontanverschlüsse um ca. 36 % während der ersten 30 min nach Substanzgabe und um 52 % zwischen der 30sten und der 60sten Minute. Von der 60sten bis zur 90sten Minute war die Verschlußrate sogar um 81 % vermindert (nicht in der Tabelle aufgeführt).

Verbindung C senkte die Verschlußhäufigkeit im ersten Beobachtungszeitraum um 55 % und im zweiten um 73 %.

Verbindung A hatte mit 1 mg/kg/h keine deutliche Wirkung, nach zusätzlichen 3 mg/kg, die jeweils nach einer lstündigen Beobachtungsperiode nach 1 mg/kg/h injiziert wurden, war die Thrombusbildung völlig aufgehoben (n = 3).

Atenolol und Propranolol wirkten mit 1 mg/kg nicht antithrombotisch.

Akute Toxizität:

Die akute Toxizität der zu untersuchenden Substanzen wurde an Mäusen (Beobachtungszeit: 14 Tage) nach oraler bzw. intravenöser Gabe bestimmt. Es wurde die $LD_{50}$ aus dem Prozentsatz der Tiere berechnet, die nach verschiedenen Dosen innerhalb der Beobachtungszeit verstarben:

| Substanz | $LD_{50}$ | |
|---|---|---|
| A | 62 mg/kg i.v. | 460 mg/kg p.o. |
| B | 75 mg/kg i.v. | 750 mg/kg p.o. |

Aufgrund ihrer antithrombotischen Eigenschaften eignen sich die Verbindungen der allgemeinen Formel I sowie ihre physiologisch verträglichen Säureadditionsalze, da diese im therapeutisch interessanten Dosisbereich eine antithrombotische Wirkung aufweisen, die nicht an die Blockade von $\beta$-Rezeptoren gebunden ist, zur Behandlung und zur Prophylaxe thromboembolischer Erkrankungen wie Coronarinfarkt, Cerebralinfarkt, sogen. transient ischaemic attacks, Amaurosis fugax, zur Prophylaxe der Arteriosklerose und zur Metastasenprophylaxe.

Die zur Erzielung einer entsprechenden Wirkung am Erwachsenen erforderliche Dosierung beträgt zweckmäßigerweise ein-bis viermal täglich 0,3 bis 4 mg/kg Körpergewicht, vorzugsweise 0,3 bis 2 mg/kg Körpergewicht. Hierzu lassen sich die erfindungsgemäß hergestellten Verbindungen der allgemeinen Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen oder Zäpfchen einarbeiten.

Die nachfolgenden Beispiele sollen die Erfindung näher erläutern:

Beispiel A

2-[4-(2-Hydroxy-3-tert.butylamino-propoxy)-phenyl]-3-methyl-6-methoxy-3,4-dihydro-chinazolin-4-on

a) 2-[4-(1,2-Epoxypropoxy)-phenyl]-3-methyl-6-methoxy-3,4-dihydro-chinazolin-4-on

2,8 g (10 mMol) 2-(4-Hydroxyphenyl)-3-methyl-6-methoxy-3,4-dihydro-chinazolin-4-on werden in 20 ml Dimethylsulfoxid gelöst und unter Rühren mit 1,35 g (10 mMol + 20 %) Kalium-tert.butylat

versetzt. Anschließend werden 2,8 ml Epibromhydrin zugegeben und bei Raumtemperatur bis zur quantitativen Umsetzung gerührt. Dann wird auf Eiswasser gegossen und das kristallin angefallene Endprodukt abgesaugt, gut mit Wasser gewaschen und getrocknet.

Schmelzpunkt: 194-196°C,

Ausbeute: 3,0 g (89 % der Theorie)

$C_{19}H_{18}N_2O_4$

| | | | | | | |
|---|---|---|---|---|---|---|
| Ber.: | C | 67,44 | H | 5,36 | N | 8,28 |
| Gef.: | | 67,41 | | 5,32 | | 8,27 |

b) 2-[4-(2-Hydroxy-3-tert.butylamino-propoxy)-phenyl]-3-methyl-6-methoxy-3,4-dihydro-chinazolin-4-on

1,7 g (5 mMol) des gemäß Beispiel 1a) erhaltenen Epoxids werden mit 17 ml tert.Butylamin in einer Stahlbombe auf 120°C erhitzt. Nach 3-stündiger Reaktionsdauer wird das überschüssige Amin im Vakuum abdestilliert und der resultierende ölige Rückstand aus Aceton/Äther umkristallisiert.

Schmelzpunkt: 154-156°C.

Ausbeute: 1,75 g (83 % der Theorie),

$C_{23}H_{29}N_3O_4$

| | | | | | | |
|---|---|---|---|---|---|---|
| Ber.: | C | 67,13 | H | 7,10 | N | 10,21 |
| Gef.: | | 67,10 | | 7,05 | | 10,19 |

Beispiel B

2-Methyl-3-[4-(2-hydroxy-3-tert.butylamino-propoxy)-phenyl]-7-methoxy-isochinolin-1(2H)-on

a) 2-Methyl-3-[4-(2,3-epoxypropoxy)-phenyl]-7-methoxy-isochinolin-1(2H)-on

2,8 g (10 mMol) 2-Methyl-3-(4-hydroxy-phenyl)-7-methoxyiso-chinolin-1(2H)-on werden in 20 ml Dimethylsulfoxid gelöst und unter Rühren mit 1,35 g (10 mMol + 20 %) Kalium-tert.butylat versetzt, wobei alsbald das Kaliumsalz ausfällt. Nun werden 2,8 ml Epibromhydrin zugegeben und bei Raumtemperatur bis zur quantitativen Umsetzung gerührt. Anschließend wird auf Eiswasser gegossen und das kristallin anfallende Endprodukt abgesaugt, gut mit Wasser gewaschen und getrocknet.

Ausbeute: 2,86 g (85 % der Theorie)

Schmelzpunkt: 153-155°C

$C_{19}H_{20}NO_4$

| | | | | | | |
|---|---|---|---|---|---|---|
| Ber.: | C | 71,20 | H | 5,68 | N | 4,15 |
| Gef.: | | 71,14 | | 5,65 | | 4,10 |

b) 2-Methyl-3-[4-(2-hydroxy-3-tert.butylamino-propoxy)-phenyl]-7-methoxy-isochinolin-1(2H)-on

2,55 g (7,5 mMol) des unter a) erhaltenen Epoxids werden mit 25 ml tert.Butylamin in einer Stahlbombe auf 120°C erhitzt. Nach 2-stündiger Reaktionsdauer ist die Umsetzung beendet und nachdem das überschüssige Amin im Vakuum abdestilliert ist, wird der resultierende körnige Rückstand aus Aceton/Äther umkristallisiert.

Ausbeute: 2,1 g (70 % der Theorie),

Schmelzpunkt: 130-131°C

$C_{24}H_{30}N_2O_4$

| | | | | | | |
|---|---|---|---|---|---|---|
| Ber.: | C | 70,22 | H | 7,32 | N | 6,82 |
| Gef.: | | 69,90 | | 7,29 | | 6,75 |

Analog den Beispielen A und B werden folgende Verbindungen hergestellt:

2-[4-[2-Hydroxy-3-(2-(3,4-dimethoxyphenyl)-N-methyl-äthylamino)-propoxy]-phenyl]-3-methyl-6-methoxy-3,4-dihydrochinazolin-4-on-dihydrochlorid

Schmelzpunkt: 152-155°C,

2-[4-[2-Hydroxy-3-(2-(2-methoxyphenyl)-äthylamino)-propoxy]-phenyl]-3-methyl-6-methoxy-3,4-dihydro-chinazolin-4-on-dihydrochlorid

Schmelzpunkt: 156-158°C,

2-[4-(2-Hydroxy-3-isopropylamino-propoxy)-phenyl]-3-methyl-6-methoxy-3,4-dihydro-chinazolin-4-on

Schmelzpunkt: 130-132°C,

2-[4-[2-Hydroxy-3-(2-(2-methylphenoxy)-äthylamino)-propoxy]-phenyl]-3-methyl-6,7-dimethoxy-3,4-dihydro-chinazolin-4-on

Schmelzpunkt: 195-198°C,

2-[4-[3-(2-Hydroxy-3-(3-methylphenoxy)-propylamino)-propoxy]-phenyl]-3-methyl-8-methoxy-3,4-dihydro-chinazolin-4-on

Schmelzpunkt:138-140°C,

2-[4-(2-Hydroxy-3-isopropylamino-propoxy)-phenyl]-3-methyl-3,4-dihydro-pyrido[2,3-e]-pyrimidin-4-on-dihydrochlorid

Schmelzpunkt des Dihydrochlorids: 142-145°C,

2-[4-(2-Hydroxy-3-tert.butylamino-propoxy)-phenyl]-3-methyl-3,4-dihydro-pyrido[2,3-e]pyrimidin-4-on-dihydrochlorid

Schmelzpunkt des Dihyrochlorids: 168-171°C,

2-[4-(2-Hydroxy-3-isopropylamino-propoxy)-phenyl]-3-methyl-3,4-dihydro-pyrido[3,4-e]-pyrimidin-4-on-dihydrochlorid

Schmelzpunkt des Dihydrochlorids: 122-125°C,

2-[4-(2-Hydroxy-3-tert.butylamino-propoxy)-phenyl]-3-methyl-3,4-dihydro-pyrido[3,4-e]pyrimidin-4-on-dihydrochlorid

Schmelzpunkt des Dihydrochlorids: 171-173°C,

2-Methyl-3-[4-(2-hydroxy-3-tert.butylamino-propoxy)-phenyl]-7-methoxy-isochinolin-1(2H)-on

Schmelzpunkt: 130-131°C,

2-Methyl-3-[2-(2-hydroxy-3-isopropylamino-propoxy)-phenyl]-7-methoxy-isochinolin-1(2H)-on-oxalat

Schmelzpunkt: 216-218°C,

2-Methyl-3-[2-(2-hydroxy-3-isopropylamino-propoxy)-4-methoxyphenyl]-7-methoxy-isochinolin-1(2H)-on-oxalat

Schmelzpunkt: 165-168°C,

2-Methyl-3-[2-(2-hydroxy-3-tert.butylamino-propoxy)-4-methoxyphenyl]-7-methoxy-isochinolin-1(2H)-on-oxalat

Schmelzpunkt:115-120°C,

2-Methyl-3-[4-(2-hydroxy-3-isopropylamino-propoxy)-phenyl]-6,7-dimethoxy-isochinolin-1(2H)-on

Schmelzpunkt: 206-207°C,

2-Methyl-3-[3-methoxy-4-(2-hydroxy-3-isopropylamino-propoxy)-phenyl]-7-methoxy-isochinolin-1(2H)-on

Schmelzpunkt: 144-146°C,

6-Methyl-7-[4-(2-hydroxy-3-tert.butylamino-propoxy)phenyl]-1,6-naphthyridin-5(6H)-on

Schmelzpunkt: 248-250°C,

6-Methyl-7-[4-(2-hydroxy-3-isopropylamino-propoxy)-phenyl]-1,6-naphthyridin-5(6H)-on

Schmelzpunkt: 135-139°C

6-Methyl-7-[4-(2-hydroxy-3-(3,4-dimethoxy-phenyl-äthyl-amino)-propoxy)-phenyl]-1,6-naphthyridin-5-(6H)-on-oxalat

Schmelzpunkt: 85-95°C.

Beispiel 1

Tabletten zu 100 mg 2-[4-(2-Hydroxy-3-tert.butylamino-propoxy)-phenyl]-3-methyl-6-methoxy-3,4-dihyro-chinazolin-4-on

Zusammensetzung:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Herstellungsverfahren:

Der Wirkstoff und der Milchzucker werden gleichmäßig mit einer wäßrigen Lösung des Polyvinylpyrrolidons befeuchtet und granuliert. Nach dem Trocknen wird das Granulat mit den restlichen Hilfsstoffen vermischt und die Mischung in üblicher Weise zu Tabletten verpreßt.

Beispiel 2

Suppositorien zu 150 mg 2-[4-(2-Hydroxy-3-tert.butylaminopropoxy)-phenyl]-3-methyl-6-methoxy-3,4-dihydro-chinazolin-4-on

Zusammensetzung:

| | |
|---|---|
| Wirkstoff | 150,0 mg |
| Suppositorienmasse | 1 550,0 mg |
| | 1 700,0 mg |

Herstellungsverfahren:

Der Wirkstoff wird in die geschmolzene Suppositorienmasse gleichmäßig eingerührt und suspendiert und das flüssige Gemisch in gekühlte Suppositorienformen ausgegossen.

Beispiel 3

Dragées zu 50 mg 2-[4-(2-Hydroxy-3-tert.butylamino-propoxy)-phenyl]-3-methyl-6-methoxy-3,4-dihydro-chinazolin-4-on

1 Dragéekern enthält:

| | |
|---|---|
| Wirksubstanz | 50,0 mg |
| Maisstärke getrocknet | 20,0 mg |
| lösliche Stärke | 2,0 mg |
| Carboxymethylcellulose | 7,0 mg |
| Magnesiumstearat | 1,0 mg |
| | 80,0 mg |

Herstellungsverfahren:

Das Gemisch wird wie in Beispiel A beschrieben zu Dragéekernen verarbeitet, die dann mit Zucker und Gummi-Arabikum dragiert werden.

Beispiel 4

Suspension mit 250 mg 2-[4-(2-Hydroxy-3-tert.butylaminopropoxy)-phenyl]-3-methyl-6-methoxy-3,4-dihydro-chinazolin-4-on pro 5 ml

100 ml Suspension enthalten:

| | | |
|---|---|---|
| Wirksubstanz | | 5,0 g |
| Carboxymethylcellulose | | 0,1 g |
| p-Hydroxybenzoesäuremethylester | | 0,05 g |
| p-Hydroxybenzoesäurepropylester | | 0,01 g |
| Zucker | | 10,0 g |
| Glycerin | | 5,0 g |
| Sorbitlösung 70 % | | 10,0 g |
| Aroma | | 0,3 g |
| destilliertes Wasser | ad | 100 ml |

Herstellungsverfahren:

In dem auf 70°C erhitzten destillierten Wasser wird unter Rühren p-Hydroxybenzoesäuremethylester und -propylester sowie Glycerin und Carboxymethylcellulose gelöst. Die Lösung wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

**Ansprüche**

1. Verwendung einer Verbindung der allgemeinen Formel

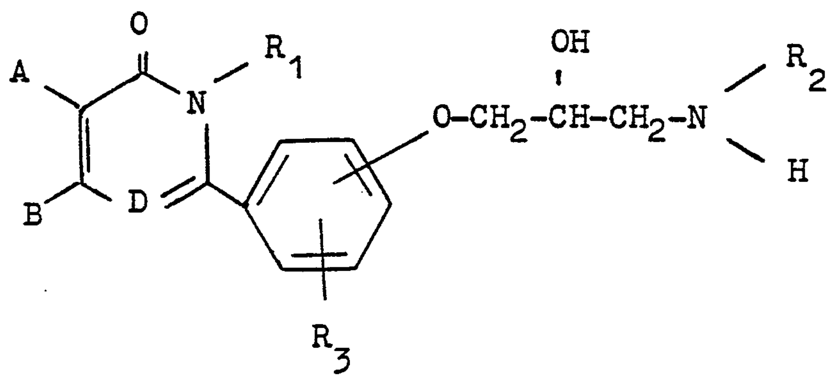

,(I)

in der

A und B zusammen mit den beiden dazwischenliegenden Kohlenstoffatomen einen Methoxyphenyl-, Dimethoxyphenyl-oder Pyridinring,

D ein Stickstoffatom oder eine CH-Gruppe,

$R_1$ ein Wasserstoffatom oder eine Methylgruppe,

$R_2$ eine verzweigte Alkylgruppe mit 3 bis 6 Kohlenstoffatomen oder eine geradkettige Alkylengruppe mit 2 oder 3 Kohlenstoffatomen, die endständig durch eine Methoxyophenyl-, Dimethoxyphenyl-oder Methylphenoxygruppe substituiert ist, und

$R_3$ ein Wasserstoffatom oder eine Methoxygruppe bedeuten, deren Antipoden und deren physiologisch verträgliche Säureadditionssalze mit anorganischen oder organischen Säuren zur Behandlung und zur Prophylaxe thrombo-embolischer Erkrankungen, zur Prophylaxe der Arteriosklerose und zur Metastasenprophylaxe.

2. Verwendung einer Verbindung der allgemeinen Formel I gemäß Anspruch 1, in der

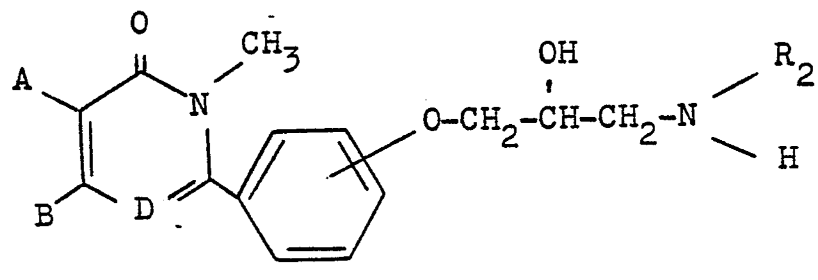

,(II)

in der

D wie im Anspruch 1 definiert ist,

A und B zusammen mit den beiden dazwischenliegenden Kohlenstoffatomen einen Methoxyphenyl-, Dimethoxyphenyl-oder Pyridinring und $R_2$ eine Isopropyl-oder tert.Butylgruppe darstellen, deren Antipoden und deren physiologisch verträgliche Säureadditionssalze mit anorganischen oder organischen Säuren zur Behandlung und zur

Prophylaxe thrombo-embolischer Erkrankungen, zur Prophylaxe der Arteriosklerose und zur Metastasenprophylaxe.

3. Verwendung einer Verbindung der allgemeinen Formel I gemäß Anspruch 2, in der

A und B zusammen mit den beiden dazwischenliegenden Kohlenstoffatomen eine

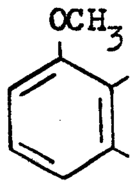,
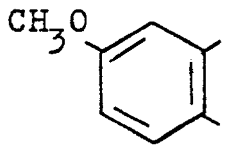,
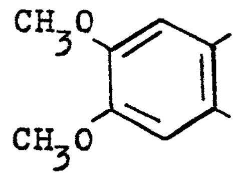
oder
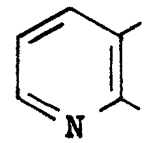
-Gruppe

bedeutet, deren Antipoden und deren physiologisch verträgliche Säureadditionssalze mit anorganischen oder organischen Säuren zur Behandlung und zur Prophylaxe thrombo-embolischer Erkrankungen, zur Prophylaxe der Arteriosklerose und zur Metastasenprophylaxe.

4. 2-Methyl-3[4-(2-hydroxy-3-tert.butylamino-propoxy)-phenyl]-7-methoxy-isochinolin-1-(2H)-on, dessen Antipoden und dessen physiologisch verträgliche Säureadditionssalze mit anorganischen oder organischen Säuren zur Behandlung und zur Prophylaxe thrombo-embolischer Erkrankungen, zur Prophylaxe der Arteriosklerose und zur Metastasenprophylaxe.

5. 3-Methyl-2-[4-(2-hydroxy-3-tert.butylamino-propoxy)-phenyl]-6-methoxy-3,4-dihydro-chinazolin-4-on, dessen Antipoden und dessen physiologisch verträgliche Säureadditionssalze mit anorganischen oder organischen Säuren zur Behandlung und zur Prophylaxe thrombo-embolischer Erkrankungen, zur Prophylaxe der Arteriosklerose und zur Metastasenprophylaxe.

6. (-)-3-Methyl-2-[4-(2-hydroxy-3-tert.butylamino-propoxy)-phenyl]-6-methoxy-3,4-dihydro-chinazolin-4-on und dessen physiologisch verträgliche Säureadditionssalze mit anorganischen oder organischen Säuren zur Behandlung und zur Prophylaxe thrombo-embolischer Erkrankungen, zur Prophylaxe der Arteriosklerose und zur Metastasenprophylaxe.

7. Verwendung einer Verbindung der allgemeinen Formel I gemäß den Ansprüchen 1 bis 6 zur Herstellung eines Arzneimittels zur Behandlung und zur Prophylaxe thrombo-embolischer Erkrankungen, zur Prophylaxe der Arteriosklerose und zur Metastasenprophylaxe.

8. Arzneimittel zur Behandlung und zur Prophylaxe thromboembolischer Erkrankungen, zur Prophylaxe der Arteriosklerose und zur Metastasenprophylaxe, enthaltend eine Verbindung der allgemeinen Formel I gemäß den Ansprüchen 1 bis 6 neben einem oder mehreren inerten Trägerstoffen und/oder Verdünnungsmitteln.

9. Verfahren zur Herstellung eines Arzneimittels gemäß Anspruch 8, dadurch gekennzeichnet, daß eine Verbindung der allgemeinen Formel I gemäß den Ansprüchen 1 bis 6 in einen oder mehrere inerte Träger-und/oder Verdünnungsmitteln auf nichtchemischem Wege eingearbeitet wird.

10. Arzneimittel gemäß Anspruch 8, dadurch gekennzeichnet, daß die Einzeldosis am Menschen 0,3 bis 4 mg/kg Körpergewicht, vorzugsweise 0,3 bis 2 mg/kg Körpergewicht, beträgt.